# EUROPEAN PATENT APPLICATION

(11) **EP 4 817 673 A1**
(43) Date of publication of application: **07.10.2026**
(21) Application number: 26168583.8
(22) Date of filing: 27.03.2026
(51) Int. Cl.: A61N 1/375

(54) **DISTAL FIXATION ASSEMBLY FOR A BIOSTIMULATOR**

(30) Priority: 31.03.2025 US 202563781088 P; 26.03.2026 US 202619630226
(71) Applicant: Pacesetter, Inc., Sylmar, CA 91342 (US)
(72) Inventor: WELCH, Mark, Sylmar, CA 91342 (US)
(74) Representative: Barker Brettell Sweden AB

(57) **Abstract**

Embodiments are disclosed of an assembly (216, 300, 400, 500, 600, 700, 750) adapted to be attached to a biostimulator (100). The assembly (216, 300, 400, 500, 600, 700, 750) includes a flange (218, 302, 402, 502, 602, 702) adapted to be attached to a distal end of the biostimulator (100). The flange (218, 302, 402, 502, 602, 702) includes a base (308, 408, 508, 608, 708) adapted to be attached to the distal end of the biostimulator (100), and a hub (310, 410, 510, 610, 710) positioned on the base (308, 408, 508, 608, 708). The hub (310, 410, 510, 610, 710) has a proximal end (311), a distal end, an interior surface (312, 412, 512, 612, 712), and an exterior surface (314, 414, 514, 614, 714). The proximal end (311) of the hub (310, 410, 510, 610, 710) is attached to the base (308, 408, 508, 608, 708), and the exterior surface (314, 414, 514, 614, 714) includes a first attachment mechanism (316, 416, 516, 616, 716). A fixation mount (220, 304, 404, 504, 604, 704) is adapted to be attached to the flange (218, 302, 402, 502, 602, 702). The fixation mount (220, 304, 404, 504, 604, 704) includes a second attachment mechanism (324, 434, 534, 624, 726) to engage with the first attachment mechanism (316, 416, 516, 616, 716). An assembly lock (417, 625, 722) engages the first attachment mechanism (316, 416, 516, 616, 716) and the second attachment mechanism (324, 434, 534, 624, 726) to fasten the flange (218, 302, 402, 502, 602, 702) to the fixation mount (220, 304, 404, 504, 604, 704).

## Description

### TECHNICAL FIELD

The disclosed embodiments relate generally to biostimulators. More specifically, but not exclusively, the disclosed embodiments relate to a distal fixation assembly used to implant biostimulators, such as leadless pacemakers, in a patient.

### BACKGROUND

Cardiac pacing by an artificial pacemaker, known more generally as a "biostimulator," provides electrical stimulation of the heart when the natural pacemaker and/or conduction system of the heart fails to provide synchronized atrial and ventricular contractions at healthy rates and intervals. Such antibradycardial pacing provides relief from symptoms, and even life support, for hundreds of thousands of patients. Cardiac pacing can also provide electrical overdrive stimulation to suppress or convert tachyarrhythmias, again supplying relief from symptoms and preventing or terminating arrhythmias that could lead to sudden cardiac death.

Currently available or conventional pacemakers usually perform cardiac pacing with an electrical pulse generator implanted away from the actual pacing site. The pulse generator is typically placed subcutaneously or sub-muscularly in or near the pectoral region of a patient and is connected to the pacing site with leads that are typically 50-70 centimeters long. The operating parameters of the pulse generator are usually interrogated and modified in one of three ways: by a programming device outside the body; via a loosely-coupled transformer with one inductance inside the body and another outside; or via electromagnetic radiation with one antenna inside the body and another outside. The implanted leads have proximal ends connected to the pulse generator and distal ends with one or more electrodes that are positioned adjacent to the inside or outside wall of a cardiac chamber. The leads have an electrical conductor for connecting the pulse generator to electrodes in the heart. Pacing leads can engage and/or be fixed to an intra-cardial implant site by an engaging mechanism such as an electrode anchor. For example, the electrode anchor can screw into the myocardium.

Leadless cardiac pacemakers locate all electronic circuitry at the pacing site, thereby eliminating electrical leads and avoiding shortcomings associated with conventional cardiac pacing systems. Leadless cardiac pacemakers can be anchored at a pacing site-for instance, in a right ventricle for single-chamber pacing or in both a right ventricle and a right atrium for dual-chamber pacing-by an anchor. A delivery system can be used to deliver the leadless cardiac pacemakers to the target anatomy and a recovery system, which in some instances can be the same as the delivery system, is used to retrieve a leadless pacemaker.

### SUMMARY

In one aspect, an assembly adapted to be attached to a biostimulator includes a flange adapted to be attached to a distal end of the biostimulator. The flange includes a base adapted to be attached to the distal end of the biostimulator, and a hub positioned on the base. The hub has a proximal end, an interior surface, and an exterior surface. The proximal end of the hub is attached to the base, and the exterior surface includes a first attachment mechanism. A fixation mount is adapted to be attached to the flange. The fixation mount includes a second attachment mechanism to engage with the first attachment mechanism. An assembly lock engages the first attachment mechanism and the second attachment mechanism to fasten the flange to the fixation mount.

In another aspect, a biostimulator includes a body having a proximal end and a distal end. A fixation assembly is adapted to be attached to the distal end of the body, and the fixation assembly includes a flange adapted to be attached to a distal end of the body. The flange includes a base adapted to be attached to the distal end of the body and a hub positioned on the base. The hub has a proximal end, an interior surface, and an exterior surface. The proximal end of the hub is attached to the base, and the exterior surface includes a first attachment mechanism. A fixation mount is adapted to be attached to the flange, and the fixation mount includes a second attachment mechanism to engage with the first attachment mechanism. A fixation element is positioned in the fixation mount, so that the flange and the fixation mount couple the fixation element to the distal end of the body. An assembly lock engages the first attachment mechanism and the second attachment mechanism to fasten the flange to the fixation mount.

In another aspect, a biostimulator system includes a catheter having a proximal end and a distal end and a biostimulator that includes a body having a proximal end and a distal end. The proximal end of the body is adapted to be removably coupled to the distal end of the catheter. A fixation assembly is attached to the distal end of the body. The fixation assembly includes a flange attached to the distal end of the body. The flange includes a base adapted to be attached to the distal end of the body, and a hub positioned on the base. The hub has a proximal end, a distal end, an interior surface, and an exterior surface. The proximal end of the hub is attached to the base, and the exterior surface includes a first attachment mechanism. A fixation mount is adapted to be attached to the flange, and the fixation mount includes a second attachment mechanism to engage with the first attachment mechanism. A fixation element is positioned in the fixation mount, and the flange and the fixation mount couple the fixation element to the distal end of the body. An assembly lock engages the first attachment mechanism and the second attachment mechanism to fasten the flange to the fixation mount.

### BRIEF DESCRIPTION OF THE DRAWINGS

Non-limiting and non-exhaustive embodiments of the present invention are described with reference to the following figures, in which like reference numerals refer to like parts throughout the various views unless otherwise specified.
FIG. 1 is a diagrammatic cross section of a human heart illustrating an embodiment of implantation of a biostimulator in a target anatomy.
FIGS. 2A-2B are perspective views of an embodiment of a biostimulator system including a biostimulator and a biostimulator transport system.
FIG. 2C is a side view of an embodiment of the distal end of a biostimulator.
FIGS. 3A-3B are views of an embodiment of a distal fixation assembly for a biostimulator.
FIGS. 4A-4D are views of another embodiment of a distal fixation assembly for a biostimulator.
FIGS. 5A-5D are views of another embodiment of a distal fixation assembly for a biostimulator.
FIGS. 6A-6B are views of another embodiment of a distal fixation assembly for a biostimulator.
FIGS. 7A-7C are views of another embodiment of a distal fixation assembly for a biostimulator.

### DETAILED DESCRIPTION

Embodiments are described of an assembly, apparatus or device, and system, for a distal fixation assembly used to implant biostimulators such as leadless pacemakers in a patient. Specific details are described to provide an understanding of the embodiments, but one skilled in the relevant art will recognize that the invention can be practiced without one or more of the described details or with other components, materials, etc. In some instances, well-known structures, materials, or operations are not shown or described in detail but are nonetheless encompassed within the scope of the invention.

Reference throughout this specification to "one embodiment" or "an embodiment" means that a described feature, structure, or characteristic can be included in at least one described embodiment, so that appearances of "in one embodiment" or "in an embodiment" do not necessarily all refer to the same embodiment. Furthermore, the described features, structures, or characteristics can be combined in any suitable manner in one or more embodiments.

As used in this application, directional terms such as "left," "right," "front," "rear," "upper," lower," "top," "bottom," "side," "lateral," "longitudinal," etc., refer to the orientations of embodiments as they are presented in the drawings, but any directional term should not be interpreted to imply or require a particular orientation of the described embodiments when in actual use. The use of relative terms throughout the description can denote a relative position or direction. For example, "distal" can indicate a first direction along a longitudinal axis of a biostimulator transport system and "proximal" can indicate a second direction opposite to the first direction. Such terms are provided to establish relative frames of reference, but are not intended to limit the use or orientation of the disclosed devices to a specific configuration described in the various embodiments below.

Delivery of a leadless pacemaker (LP) may rely on applying a torque to the LP to fix a fixation element, e.g., a fixation helix, into the target tissue. This rotation that supplies torque to the LP can be isolated from the catheter's deflection, so that the torque and linear motion do not affect each other-i.e., torque does not affect linear motion and linear motion does not affect torque. Isolating the rotation and torque supply ensures that the catheter can be deflected to put the LP in the correct position and can maintain the correct position while the LP is rotated to fixate the helix in the target location. Existing assemblies at the distal end of a catheter use a manufacturer's existing bearing that is captured in place to isolate torque from the catheter. But there is limited space available within the right ventricle and atrium, meaning that a reduction in rigid length-i.e., the length of rigid components at the distal end of the catheter-is desirable to promote better catheter steerability. This application discloses embodiments of distal bearing assemblies that reduce rigid length to improve a range of deflection of the catheter and ability to be positioned.

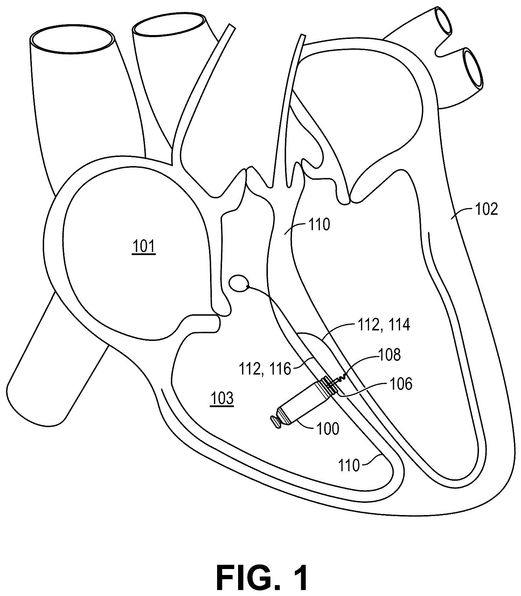

FIG. 1 illustrates an embodiment of implantation of a biostimulator in a target anatomy of a human heart. A leadless biostimulator system, e.g., a cardiac pacing system, includes one or more biostimulators 100 that can be implanted in a patient heart 102 and can be leadless (e.g., it can be a leadless pacemaker (LP)). Each biostimulator can be placed in a cardiac chamber, such as a right atrium 101 and/or right ventricle 103, or attached to an inside or outside of the cardiac chamber. For example, biostimulator 100 can be attached to a septum 110 of heart 102. More particularly, biostimulator 100 can be delivered to the septum 110, and one or more elements, such as a fixation element 106 and/or a pacing element 108, can pierce septal wall 110 to engage and anchor the biostimulator 100 to the target anatomy, e.g., a bundle branch 112 in the septal wall 110. In a particular embodiment, biostimulator 100 can use two or more electrodes located on or within a housing of the biostimulator 100 for pacing the cardiac chamber upon receiving a triggering signal from at least one other device within the body. In an embodiment, one or more of the fixation element 106 or the pacing element 108 is an active electrode.

When biostimulator 100 is delivered to and screwed into the target tissue-e.g., an atrial or ventricular wall, or a septum 110 of the heart 102-pacing element 108 and/or fixation element 106 can be positioned for pacing. For example, in the case of deep septal pacing at bundle branches 112 in septum 110, an active electrode of pacing element 108 can be positioned at a first target anatomy in the septal wall 110, e.g., a left bundle branch 114. Similarly, the fixation element 106 can be positioned at a second target anatomy in the septal wall, e.g., a right bundle branch 116. Optionally, one of the elements can be at a bundle branch and the other element need not be at a bundle branch.

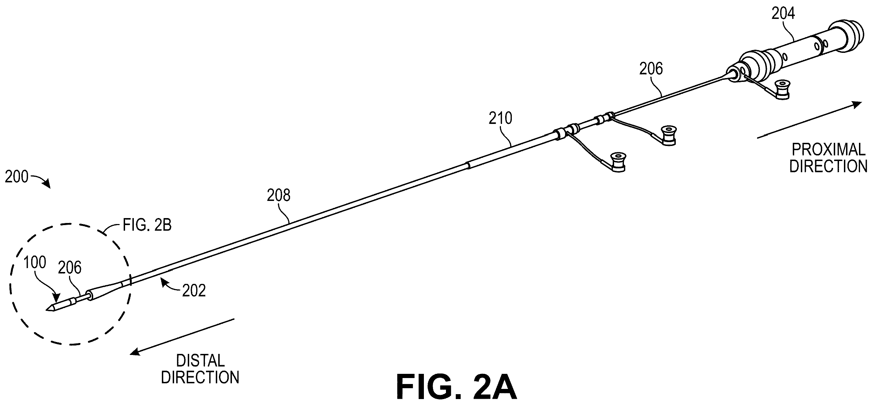

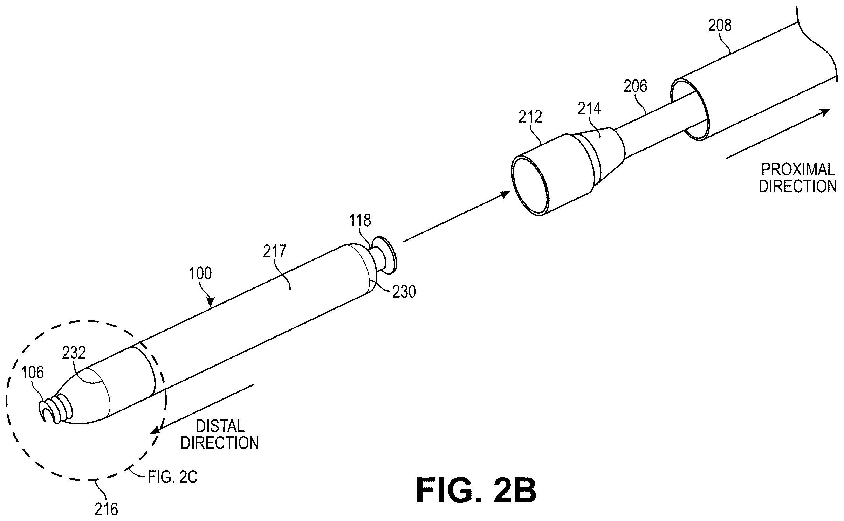

FIGS. 2A-2B together illustrate an embodiment of a biostimulator system 200. FIG. 2A is a perspective view of the biostimulator system 200, and FIG. 2B is a perspective view of a distal end of the biostimulator system 200. Biostimulator system 200 can include a biostimulator 100, e.g., a leadless pacemaker or other leadless biostimulator. Biostimulator system 200 can also include delivery or retrieval systems, which can be catheter-based systems used to carry biostimulator 100 intravenously to or from a patient anatomy. For example, a biostimulator transport system 202 can be used to deliver biostimulator 100 to, or retrieve the biostimulator from, a patient. Biostimulator 100 can be attached, connected to, or otherwise mounted on biostimulator transport system 202. For example, the biostimulator 100 can be mounted on a distal end of the biostimulator transport system 202. In an embodiment, the biostimulator transport system 202 includes a catheter 206 extending longitudinally to a docking cap 212 at the distal end. The biostimulator 100 can include a body 217 having a proximal end 230 and a distal end 232. In an embodiment, an attachment feature 118 may be coupled to the proximal end 230 of the body 217. The attachment feature 118 of the biostimulator 100 can be received and/or mounted in the docking cap 212 (FIG. 2B). The biostimulator transport system 202 can then be used to advance biostimulator 100 intravenously into or out of heart 102.

Biostimulator transport system 202 can include a handle 204 to control movement and operations of the biostimulator transport system 202 from outside a patient. One or more elongated members extend distally from handle 204. For example, an elongated catheter body 206 can extend distally from handle 204 to a distal end of the biostimulator transport system 202. In an embodiment, biostimulator 100 is mounted on a distal end of elongated catheter body 206.

Biostimulator transport system 202 can include a protective sleeve 208 to cover biostimulator 100 during delivery and implantation. Protective sleeve 208 can extend over, and be longitudinally movable relative to, elongated catheter body 206. The biostimulator transport system 202 can also include an introducer sheath 210 that can extend over, and be longitudinally movable relative to, the protective sleeve 208. Introducer sheath 210 can cover a distal end of the protective sleeve 208, the elongated catheter body 206, and biostimulator 100, as those components are passed through an access device into the patient anatomy.

Biostimulator transport system 202 can also be configured to include additional or alternate components. More particularly, the biostimulator transport system 202 can be configured to deliver biostimulator 100 to, or retrieve it from, the target anatomy. Delivery and/or retrieval of biostimulator 100 can include retaining the biostimulator 100 on catheter 206 during transport to the target anatomy and rotation of the biostimulator 100 during its implantation at the target anatomy. Accordingly, biostimulator transport system 202 can incorporate features to retain and rotate biostimulator 100, including at least those described below.

FIG. 2B illustrates an embodiment of a distal portion of a biostimulator transport system 202 and its attachment to a biostimulator 100. The view is a close-up view of a distal portion of the biostimulator transport system 202 shown in FIG. 2A. The biostimulator transport system 202 can include features to engage the biostimulator 100 and to allow screwing the biostimulator 100 into the target tissue.

Similar to the retrieval system, the delivery system can include the docking cap 212 having a key configured to engage attachment feature 118 of the biostimulator 100 and apply torque to screw the fixation element 106 into the target tissue. The biostimulator transport system 202 may also include the elongated catheter 206 having a torque shaft on which docking cap 212 and bearing housing 214 are mounted. The protective sheath 208 can be positioned along the outer catheter 206, and can be advanced or retracted to cover or expose the docking cap 212 and biostimulator 100.

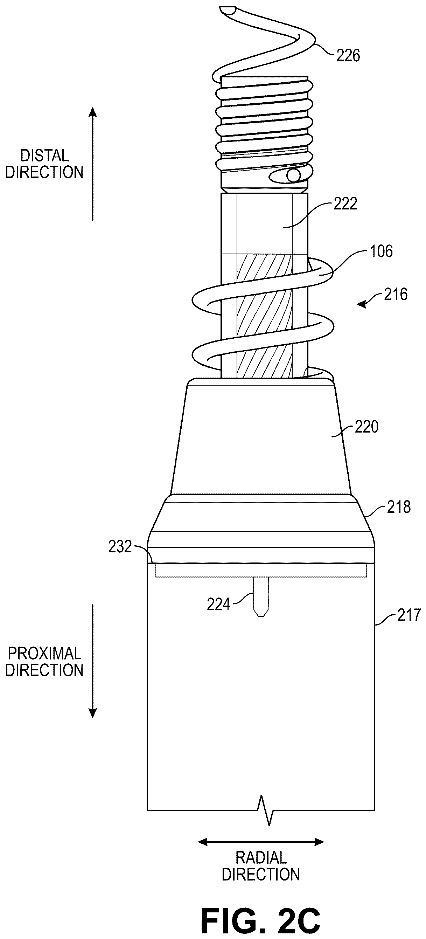

FIG. 2C illustrates an embodiment of a distal fixation assembly 216 for biostimulator 100-i.e., the area highlighted in a dashed circle in FIG. 2B. The distal end assembly 216 can include one or more components attached to the distal end 232 of body 217 of biostimulator 100. The component(s) can include a flange 218, a fixation mount 220, a fixation element 106, or a lead body 222. For example, the lead body 222 can be centrally positioned in one or more of the flange 218, the fixation mount 220, or the fixation element 106.

Flange 218 can be connected directly to the distal end 232 of body 217 and can include a feedthrough-e.g., a hole or other void in one embodiment-through which an electrical connection can be formed between pacing element 226 and electrical circuitry within biostimulator 100. In the illustrated embodiment, a pin 224 within body 217 runs in a distal direction from the body 217, through the feedthrough in the flange 218, and is then electrically mechanically coupled to lead body 222 and/or electronically coupled to pacing element 226. Pin 224 and pacing element 226 thus form the electrical path through which biostimulator 100 transmits electrical signals to the target tissue being paced. Embodiments of flanges that can be used as flange 218 are further discussed below.

Fixation mount 220 surrounds lead body 222 and is attached to flange 218. Fixation element 106 is in turn attached to fixation mount 220, so that fixation mount 220 and flange 218 together attach fixation element 106 to biostimulator 100. Embodiments of digital fixation assemblies 216, including fixation mounts 220 and their connection to the flange 218 and fixation element 106, are further discussed below.

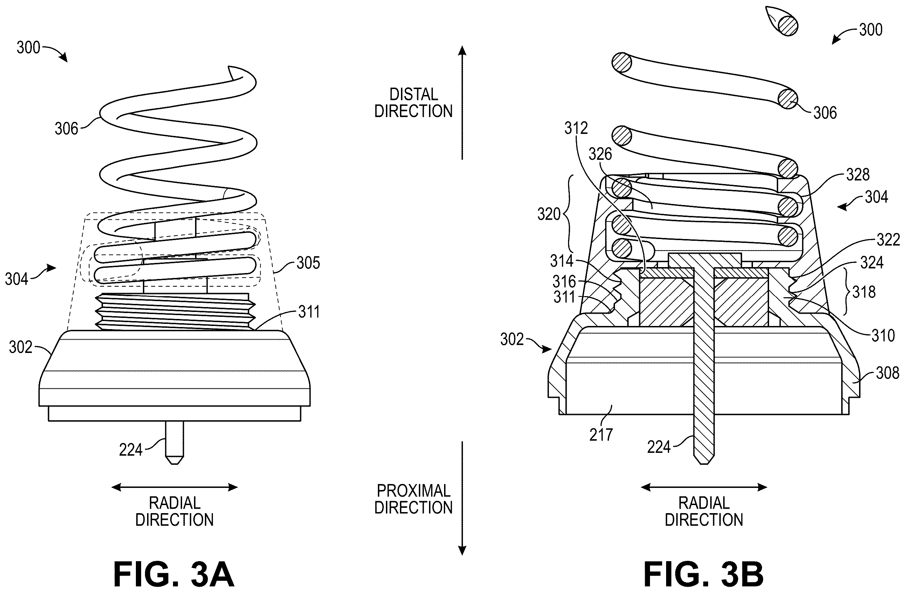

FIGS. 3A-3B together illustrate an embodiment of a distal fixation assembly 300 for a biostimulator 100. FIG. 3A is a side view and FIG. 3B is a sectional side view. Distal fixation assembly 300 can include one or more components that can be attached, as shown in FIG. 2C, to the distal end 232 of body 217 of a biostimulator 100. The component(s) can include a flange 302, a fixation mount 304, or a fixation element 306. Lead body 222 is not shown in this embodiment but can nonetheless be included as shown in FIG. 2C: and then preferably centrally positioned in one or more of the flange 302, the fixation mount 304, or the fixation element 306.

Flange 302 is connected to the distal end 232 of body 217 and includes a feedthrough-e.g., a hole or other void in one embodiment-through which an electrical connection can be formed between pacing element 226 (see e.g., FIG. 2C) when present, and electrical circuitry within biostimulator 100. Flange 302 includes a base 308 and a hub 310 that surrounds and forms the feedthrough. In the illustrated embodiment hub 310 is centrally positioned on base 308, but in other embodiments it need not be centrally positioned. Hub 310 can have a proximal end 311, an interior surface 312, and an exterior surface 314. Interior surface 312 is substantially at a first radial position and exterior surface 314 is substantially at a second radial location greater than the first radial location, so that the material between interior surface 312 and exterior surface 314 forms a sidewall of the hub 310. Interior surface 312 forms a hub lumen that forms the feedthrough. A first attachment mechanism, such as a first set of threads 316, is formed on exterior surface 314 so that fixation mount 304 can be attached to hub 310 and thus to flange 302.

Fixation mount 304 is adapted to be attached to both flange 302 and fixation element 306. In the illustrated embodiment, the fixation mount 304 has an exterior surface 305 of frustoconical shape, but in other embodiments exterior surface 305 can have a different shape than shown. In one embodiment the fixation mount 304 can be made of polyetheretherketone (PEEK), but in other embodiments it can be made of a different material. The interior of fixation mount 304 has two main parts: a proximal part 318 that receives hub 310 and a distal part 320 that receives fixation element 306. Proximal part 318 includes an interior surface 322. The interior surface 322 can have a second fixation mechanism, such as a second set of threads 324, formed therein. Second set of threads 324 complements first set of thread 316-i.e., attributes of both sets of threads 316, 324, such as their pitch, are matched so that second threads 324 will mesh with first threads 316. Using first threads 316 and second threads 324, fixation mount 304 can be attached to flange 302 by screwing the fixation mount onto hub 310. In one embodiment, the distal fixation assembly 300 can include an assembly lock that prevents the fixation mount 304 from rotating relative to the flange 302 after the first set of threads 316 engages the second set of threads 324.

Various assembly locks, e.g., thread locking features, may be incorporated into the distal fixation assembly 300. The assembly lock can engage the first attachment mechanism and the second attachment mechanism to fasten the flange 302 to the fixation mount 304. In an embodiment, a medical grade adhesive is placed between the threads, i.e., the first set of threads 316 and the second set of threads 324, to adhere the thread surfaces. More particularly, the assembly lock can include a glue or an adhesive, e.g., a threadlocker adhesive, to attach the fixation mount 304 to the flange 302.

In an alternative embodiment, the thread locking feature includes a thread geometry that allows one set of threads to bind with the adjacent structure. For example, the threads of the fixation mount 304 may have a different thread width than the threads of the flange 302. The threads may therefore form an interference fit. The threads can press tightly against each other, causing the structures to bind and resist relative rotation. Accordingly, an undersized thread callout of one set of threads can generate a force fit that locks the threads and attaches the fixation mount 304 to the flange 302.

The thread locking feature may alternatively include a thread geometry that causes one group of threads to tap into the adjacent structure. For example, a length of the first set of threads 316 can be longer than a length of the second set of threads 324. When the fixation mount 304 is screwed onto the flange 302, the threads may reach a point at which the end of one thread is reached, while the other thread has additional length that can be screwed into place. At that point, an assembler can continue to torque the components together, causing the longer thread to tap into the surface of the adjacent structure, e.g., cutting a thread form into the material of the adjacent structure. The cut threads can press tightly against the contacting surface having the longer thread, and the sets of threads may therefore be locked together. A structural fingerprint of such an assembly is that the shorter thread will have two regions, a preformed thread region and a tapped thread region, having different thread widths. More particularly, the preformed thread region can have a narrower thread width to receive the longer thread, and the tapped thread region can have a wider thread width because it will have no additional relief but will instead fill an entire space between adjacent thread turns of the longer thread.

Distal part 320 of the interior of fixation mount 304 has an interior surface 326 in which is formed a helical groove 328. Helical groove 328 receives fixation element 306, which may include a helically formed wire or other helical anchor, and the attributes of helical groove 328, such as its pitch, are matched to the pitch of the fixation element 306 so that the fixation element 306 can be threaded into the helical groove 328. Once threaded into the helical groove 328, the fixation element 306 is secured to the interior of the fixation mount 304. By attaching fixation element 306 to fixation mount 304 and attaching fixation mount 304 to hub 310, the fixation element 306 is attached to flange 302 and to biostimulator body 217.

In the illustrated embodiment, a pin 224 within body 217 runs in a distal direction from the body 217 through the lumen of hub 310, and is then electrically coupled to pacing element 226 (see FIG. 2C) when present. Pin 224 and pacing element 226 thus form an electrical path through which biostimulator 100 can transmit electrical signals to the target tissue being paced.

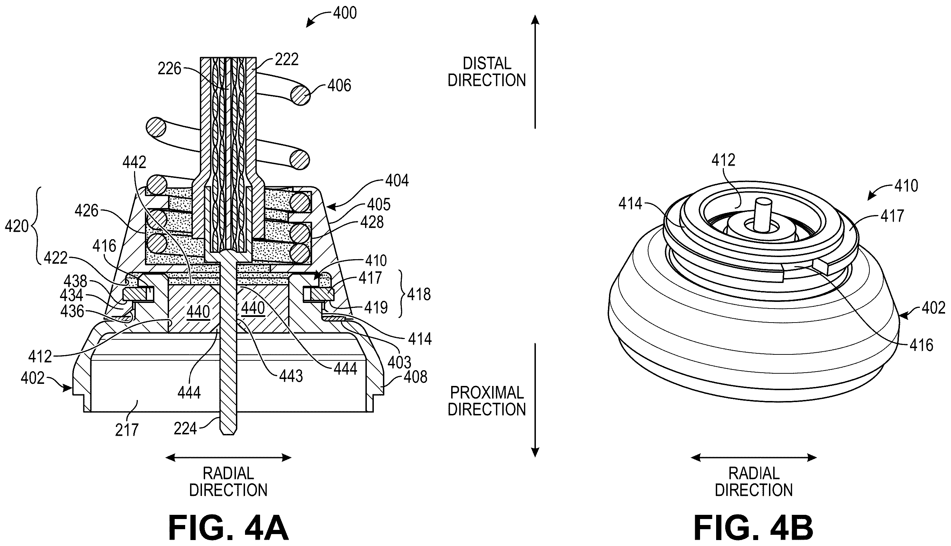

FIGS. 4A-4D together illustrate another embodiment of a distal fixation assembly 400 for a biostimulator 100. Distal fixation assembly 400 can include one or more components. The component(s) can include: a flange 402, a fixation mount 404, or a fixation element 406. As in previous embodiments, lead body 222 may be centrally positioned in one or more of the flange 402, the fixation mount 404, or the fixation element 406.

Flange 402 is connected to the distal end 232 of body 217 and includes a feedthrough-e.g., a hole or other void in one embodiment-through which an electrical connection can be formed between pacing element 226, when present, and electrical circuitry within biostimulator 100. Flange 402 includes a base 408 and a hub 410 that surrounds and forms the feedthrough. In the illustrated embodiment hub 410 is centrally positioned on base 408, but in other embodiments it need not be centrally positioned. Hub 410 includes an interior surface 412 and an exterior surface 414. Interior surface 412 is substantially at a first radial position and exterior surface 414 is substantially at a second radial location greater than the first radial location, so that the material between interior surface 412 and exterior surface 414 forms a sidewall of the hub 410. Interior surface 412 forms a hub lumen in which the feedthrough is formed.

A groove 416 is formed in exterior surface 414 of the hub 410. In the illustrated embodiment, groove 416 goes around the entire circumference of exterior surface 414, but in other embodiments it need not go around the entire circumference. The groove 416 can be a first attachment mechanism used to receive an assembly lock. More particularly, the assembly lock can include a retaining ring 417, and the retaining ring 417 may be mounted in the groove 416. To get retaining ring 417 into groove 416, the retaining ring 417 is preferably flexible and can be expanded radially outward until its inner diameter is at least the diameter of outer surface 414, for instance applying a radially outward force. When expanded, the flexible retaining ring 417 can then be slipped in a proximal direction onto the hub 410 until its position coincides with groove 416, at which point the radial force on the retaining ring 417 is released and, due to its flexibility, the retaining ring 417 snaps into the groove 416. When is positioned in groove 416, retaining ring 417 projects radially beyond outer surface 414, forming a first lip with a proximally-facing surface 419 (i.e., a lip with a surface whose normal vector points substantially in a proximal direction or has a non-zero component in the proximal direction). In one embodiment, retaining ring 417 is a C-ring, but in other embodiments it can be another type of ring. In one embodiment retaining ring 417 can be made of a flexible material such as MP35NLT, but in other embodiments it can be made of a different material or combination of materials.

Fixation mount 404 is adapted to be attached to both flange 402 and fixation element 406. In the illustrated embodiment, fixation mount 404 has an exterior surface 405 of frustoconical shape, but in other embodiments, exterior surface 405 can have a different shape than shown. The interior of fixation mount 404 has two main parts: a proximal part 418 that receives hub 410 and retaining ring 417, and a distal part 420 that receives fixation element 406.

Proximal part 418 of the interior of fixation mount 404 includes an interior surface 422 with a second lip 434 formed thereon. Second lip 434 complements first lip 419-i.e., the lips are matched so that second lip 434 will engage first lip 419. The second lip 434 can provide an attachment mechanism to engage the first attachment mechanism provided by the groove 416. More particularly, the second attachment mechanism can include a distally-facing lip 434, which can latch onto the retaining ring 417 in the groove 416. In the illustrated embodiment, second lip 434 includes at least a distally-facing surface 438 (i.e., a surface whose normal vector points substantially in a distal direction or has a non-zero component in the distal direction). Second lip 434 can also include a proximally slanted surface 436 (i.e., a surface whose normal vector has non-zero proximal and radial components). Slanted surface 436 can radially compress the retaining ring 417, making it easier to attach fixation mount 404 to hub 410. In other embodiments second lip 434 can be configured differently than shown.

Distal part 420 of the interior of fixation mount 404 has an interior surface 426 in which is formed a helical groove 428. Helical groove 428 receives fixation element 406, and the attributes of helical groove 428, such as its pitch, are matched to the pitch of the fixation element 406 so that the fixation element 406 can be threaded into the helical groove 428. Once threaded into the helical groove 428, fixation element 406 is secured to the interior of the fixation mount 404. By attaching fixation element 406 to fixation mount 404 and attaching fixation mount 404 to hub 410, the fixation element 406 is attached to flange 402 and biostimulator body 217. If after fixation mount 404 is attached to flange 402 there remains a gap between the fixation mount 404 and the flange 402, a seal 403 can be positioned between the two to prevent fluid intrusion.

In the illustrated embodiment, a feedthrough in the lumen of hub 410 includes an electrically insulating disk 440, which in one embodiment can be a ceramic material but in other embodiments can be a different kind of electrically-insulating material. Disk 440 includes a centrally-positioned disk hole 443 with tapered proximal and distal ends. Pin 224 runs distally from body 217 through disk hole 443 and is electrically coupled to pacing element 226, forming an electrical path through which biostimulator 100 transmits electrical pulses to the target tissue being paced. The pin 224 can be held in place, and the feedthrough sealed against intrusion of fluids, with a material 444 inserted or deposited into the tapered ends of disk hole 443. In one embodiment, material 444 can be gold that is reflowed into the tapered ends, but other embodiment can use a different material. An epoxy cap 442 can be placed over the lumen of hub 410 to provide further protection and sealing. In the illustrated embodiment the epoxy cap extends radially to the outer surface 414 of hub 410, but in other embodiments it need not extend that far radially.

As shown in FIG. 4A, epoxy may fill a cavity within the fixation mount 404. For example, the speckled hatching can represent an epoxy fill material that fills the cavity and surrounds the internal structures such as the fixation element 406. The epoxy may both bond and seal the components of the distal fixation assembly 400 in place. More particularly, the epoxy can form a hermetic seal surrounding the electrical feedthrough and electrode components within the fixation mount 404.

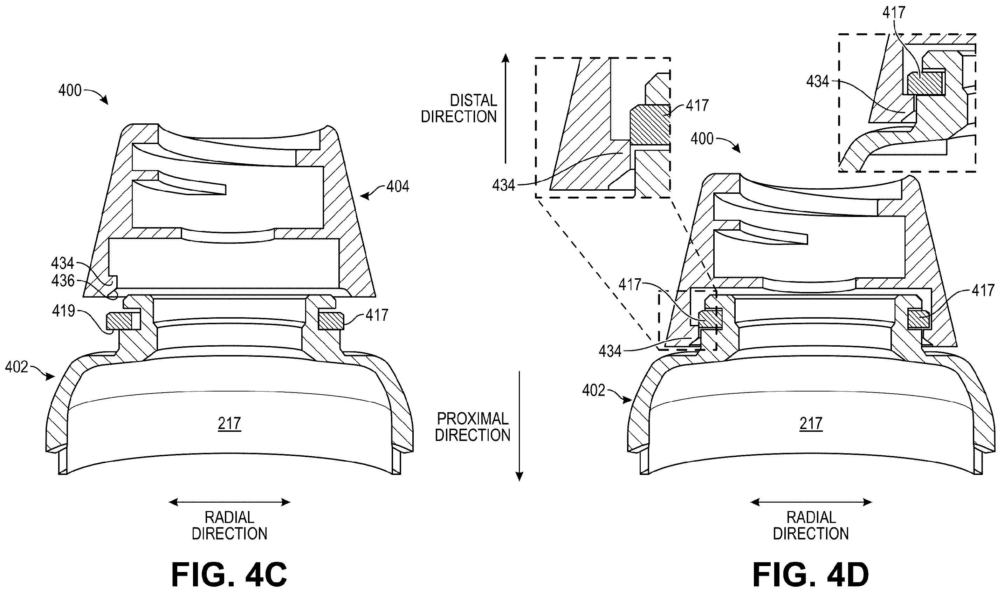

FIGS. 4C-4D, and the insets in FIG. 4D, illustrate how fixation mount 404 can be attached to the flange 402. The fixation mount 404 can be attached to flange 402 by pressing fixation mount 404 proximally (i.e., in a proximal direction) onto hub 410. When pressed proximally onto hub 410, slanted surface 436 first engages with retaining ring 417. As fixation mount 404 is pushed proximally, it deflects radially outward and retaining ring 417 is compressed radially inward. Fixation mount 404 is further pushed proximally until second lip 434 moves proximally past retaining ring 417, as which point second lip 434 snaps radially inward and the retaining ring 417 snaps radically outward so that distally-facing surface 438 engages the retaining ring 417, e.g., the proximally-facing surface 419, securing fixation mount 404 onto hub 410 and thus onto the base 408 of the flange 402.

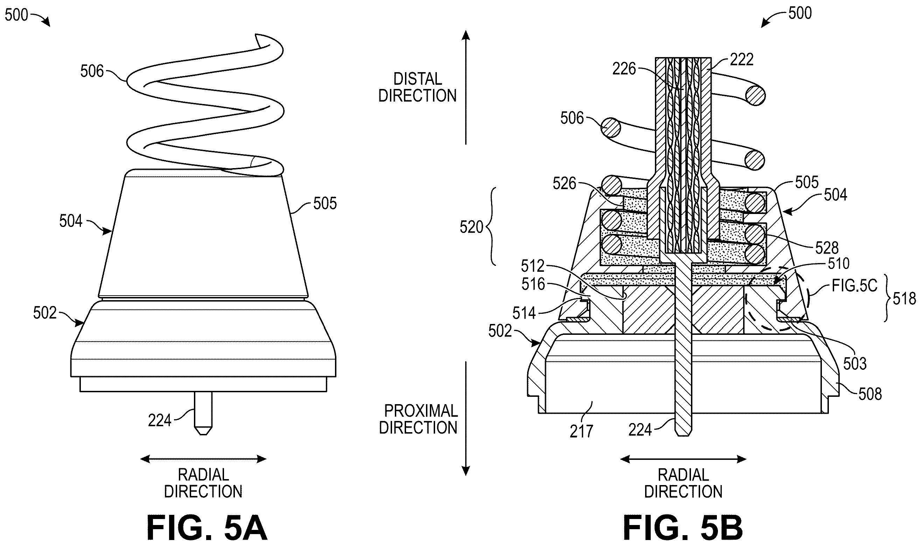

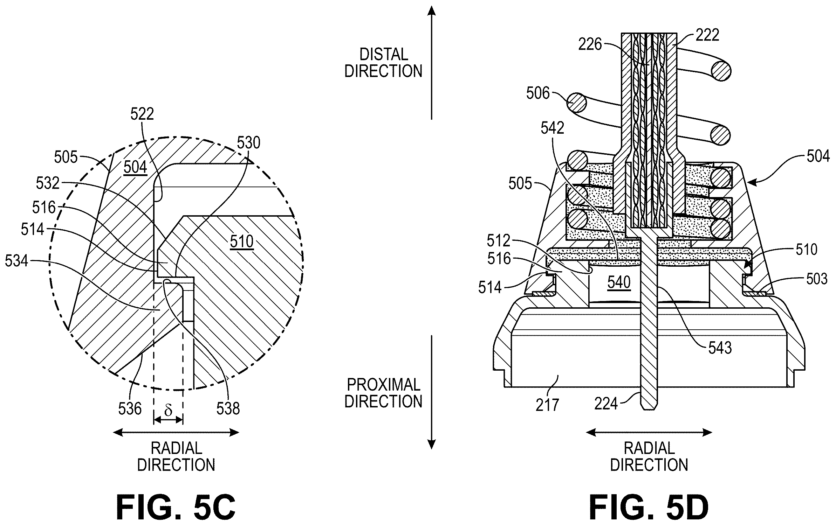

FIGS. 5A-5C together illustrate another embodiment of a distal fixation assembly 500 for a biostimulator 100. FIG. 5A is a side view, FIG. 5B is a sectional side view, and FIG. 5C is an enlarged sectional view of a portion of FIG. 5B. Distal fixation assembly 500 can includes one or more components. The component(s) can include a flange 502, a fixation mount 504, or a fixation element 506. Lead body 222 may be centrally positioned in the flange 502, the fixation mount 504, or the fixation element 506.

Flange 502 is connected to the distal end 232 of body 217 and includes a feedthrough-e.g., a hole or other void in one embodiment-through which an electrical connection can be formed between pacing element 226 and electrical circuitry within biostimulator 100. Flange 502 includes a base 508 and a hub 510 that surrounds and forms the feedthrough. In the illustrated embodiment hub 510 is centrally positioned on base 508, but in other embodiments it need not be centrally positioned. Hub 510 has an interior surface 512 and an exterior surface 514. Interior surface 512 is substantially at a first radial position and exterior surface 514 is substantially at a second radial location greater than the first radial location, so that the material between interior surface 512 and exterior surface 514 forms a sidewall of the hub. Interior surface 512 forms a hub lumen in which the feedthrough is formed.

In an embodiment, a first attachment mechanism of the assembly includes a first lip 516. The first lip 516 is formed on exterior surface 514 of the hub 510. In the illustrated embodiment, lip 516 includes at least a distally slanted surface 532 (i.e., a surface whose normal vector has non-zero distal and radial components) and a proximally-facing surface 530 (i.e., a surface whose normal vector points substantially in a proximal direction or has a non-zero component in the proximal direction). In other embodiments first lip 516 can be configured or structured differently than shown.

Fixation mount 504 is adapted to be attached to both flange 502 and fixation element 506. In the illustrated embodiment, fixation mount 504 has an exterior surface 505 of frustoconical shape, but in other embodiments exterior surface 505 can have a different shape than shown. The interior of fixation mount 504 is divided into two parts: a proximal part 518 that receives hub 510, and a distal part 520 that receives fixation element 506. Proximal part 518 of the interior of fixation mount 504 includes an interior surface 522 (see FIG. 5C) with a second lip 534 formed thereon. The second lip 534 can provide a second attachment mechanism. More particularly, the second lip 534 can complement first lip 516-i.e., the lips are matched so that second lip 534 will engage first lip 516. In the illustrated embodiment, second lip 534 includes at least a proximally slanted surface 536 (i.e., a surface whose normal vector has non-zero proximal and radial components) and a distally-facing surface 538 (i.e., a surface whose normal vector points substantially in a distal direction or has a non-zero component in the distal direction). In other embodiments second lip 534 can be configured differently than shown.

Using first lip 516 and second lip 524, fixation mount 504 can be attached to flange 502 by pressing fixation mount 504 proximally (i.e., in a proximal direction) onto hub 510. When pressed proximally onto hub 510, slanted surface 536 first engages with slanted surface 532. As fixation mount 504 is pushed proximally, it deflects radially outward; dimension δ of the second lip 534 (see Fig. 5C) is chosen so that the outward radial deflection will remain within the plastic deformation range of the material fixation mount 504 is made of. Fixation mount 504 is further pushed proximally until second lip 534 moves proximally past proximally-facing surface 530, as which point second lip 534 snaps radially inward so that distally-facing surface 538 engages proximally-facing surface 530 to form an assembly lock. The assembly lock, formed by the engagement of the lips 516, 524 of the attachment mechanisms, can secure fixation mount 504 onto hub 510 and thus onto flange 502.

Distal part 520 of the interior of fixation mount 504 has an interior surface 526 in which is formed a helical groove 528. Helical groove 528 receives fixation element 506, and the attributes of helical groove 528, such as its pitch, are matched to the pitch of fixation element 506, so that the fixation element 506 can be threaded into the helical groove 528. Once threaded into the helical groove 528, the fixation element 506 is secured to the interior of the fixation mount 504. By attaching fixation element 506 to fixation mount 504, and attaching fixation mount 504 to flange 510, the fixation element 506 is attached to flange 502 and biostimulator body 217. If after fixation mount 504 is attached to flange 502 there remains a gap between the fixation mount 504 and the flange 502, a seal 503 can be positioned between the two to prevent fluid intrusion.

FIGS. 5B and 5D illustrate embodiments of feedthroughs. In both embodiments a feedthrough is positioned in the lumen of hub 510. In the embodiment of FIG. 5B, the feedthrough can be configured similarly to the feedthrough described above for distal fixing assembly 400. In the embodiment of FIG. 5D, a glass or glasslike disk 540 is positioned in the lumen of hub 510. The disk 540 includes a centrally-positioned disk hole 543. Pin 224 runs in a distal direction from body 217 through disk hole 543 and is electrically coupled to pacing element 226, forming an electrical path through which biostimulator 100 can transmit electrical pulses to the target tissue being paced. The pin 224 can be held in place, and the feedthrough sealed against intrusion of fluids, by heating glass disk 540-i.e., heating to a temperature where it begins to expand, then cooling it again until it contracts. When expanded, the glass or glasslike material seals gaps between the disk 540 and pin 224 and gaps between the disk 540 and inner surface 512. An epoxy cap 542 can be placed over the lumen of hub 510 to provide further protection and sealing. In the illustrated embodiment epoxy cap 542 extends radially to the outer surface of hub 510, but in other embodiments it need not extend that far radially.

As shown in FIGS. 5B and 5D, epoxy may fill a cavity within the fixation mount 504. For example, the speckled hatching can represent an epoxy fill material that fills the cavity and surrounds the internal structures such as the fixation element 506. The epoxy may both bond and seal the components of the distal fixation assembly 500 in place. More particularly, the epoxy can form a hermetic seal surrounding the electrical feedthrough and electrode components within the fixation mount 504.

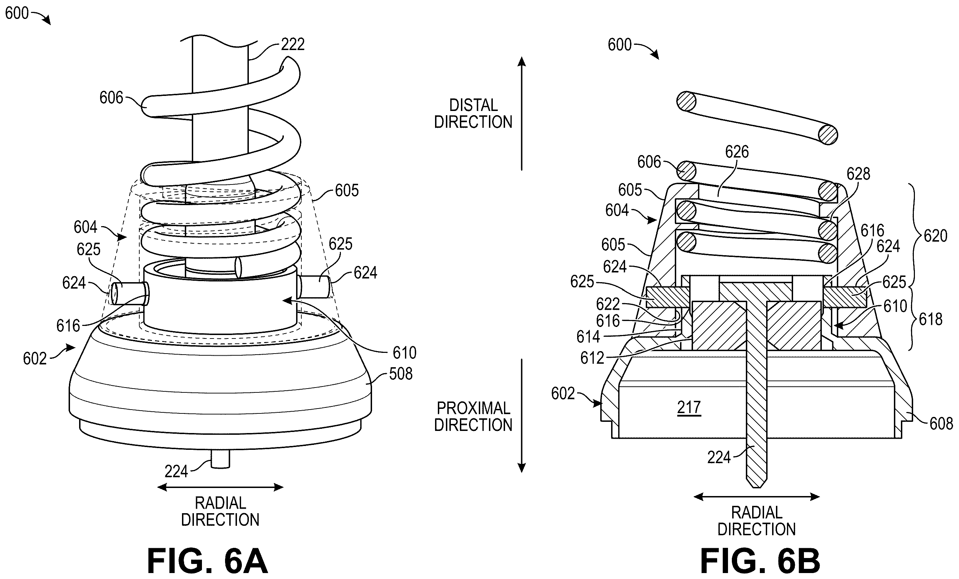

FIGS. 6A-6B together illustrate another embodiment of a distal fixation assembly 600 for a biostimulator 100. FIG. 6A is a side view, FIG. 6B a sectional side view. Distal fixation assembly 600 can includes one or more components. The component(s) can include a flange 602, a fixation mount 604, or a fixation element 606. As in previous embodiments, lead body 222 may be centrally positioned in flange 602, the fixation mount 604, and the fixation element 606.

Flange 602 is connected to the distal end 232 of body 217 and includes a feedthrough-e.g., a hole or other void in one embodiment-through which an electrical connection can be formed between pacing element 226, when present, and electrical circuitry within biostimulator 100. Flange 602 includes a base 608 and a hub 610 that surrounds and forms the feedthrough. In the illustrated embodiment hub 610 is centrally positioned on base 608, but in other embodiments it need not be centrally positioned. Hub 610 has an interior surface 612 and an exterior surface 614. Interior surface 612 is substantially at a first radial position and exterior surface 614 is substantially at a second radial location greater than the first radial location, so that the material between interior surface 612 and exterior surface 614 forms a sidewall of the hub 610. Interior surface 612 forms a hub lumen in which the feedthrough is formed.

A pair of radial holes 616 are formed in the sidewall of hub 610. The holes 616 can provide a fixation mechanism. For example, radial holes 616 formed in the exterior sidewall of the hub 610 can provide a first fixation mechanism. In the illustrated embodiment the radial holes 616 extend through the entire thickness of the hub sidewall, from exterior surface 614 to interior surface 612, but in other embodiment holes 616 need not extend through the entire sidewall thickness. Also the illustrated embodiment has two radial holes 616 and they are diametrically opposed, but in other embodiments there can be more or less radial holes and, in embodiments with multiple holes, they need not be diametrically opposed nor uniformly distributed around the circumference of the hub 610.

Fixation mount 604 is adapted to be attached to both flange 602 and fixation element 606. In the illustrated embodiment, fixation mount 604 has an exterior surface 605 of frustoconical exterior shape, but in other embodiments exterior surface 605 can have a different exterior shape than shown. The interior of fixation mount 604 has two parts: a proximal part 618 that receives hub 610, and a distal part 620 that receives fixation element 606.

Proximal part 618 of the interior of fixation mount 604 includes an interior surface 622 that is shaped and dimensioned to substantially contact exterior surface 614 of hub 610. Fixation mount 604 includes radial holes 624 that are positioned so that they will align with holes 616 in hub 610. Generally, each hole 624 in fixation mount 604 will have a corresponding hole 616 in hub 610, but that need not be the case in every embodiment. As in hub 610, the holes 624 extend through the entire thickness of the sidewall of fixation mount 604. Also, the illustrated embodiment has two holes 624 and they are diametrically opposed, but in other embodiments there can be more or less holes and, in embodiments with multiple holes, they need not be diametrically opposed nor uniformly distributed around the circumference of the hub 610. The holes 624 in the fixation mount 604 can provide a second fixation mechanism. More particularly, the second attachment mechanism can include a radial hole 624 formed in the fixation mount 604. In an embodiment, an assembly lock includes a pin 625. The pin 625 can be is inserted in at least one hole pair-i.e., a hole 624 and its corresponding hole 616-to attach fixation mount 604 to hub 610. In different embodiment pin(s) 625, once inserted, can be secured in place in different ways: adhesives, threading, or an interference fit between the pin(s) 625 and holes 616, 624 are examples.

Distal part 620 of the interior of fixation mount 604 has an interior surface 626 in which is formed a helical groove 628. Helical groove 628 receives fixation element 606, and the attributes of helical groove 628, such as its pitch, are matched to the pitch of fixation element 606, so that the fixation element 606 can be threaded into the helical groove 628. Once threaded into the helical groove 628, the fixation element 606 is secured to the interior of the fixation mount 604. By attaching fixation element 606 to fixation mount 604, and attaching fixation mount 604 to hub 610, the fixation element 606 is attached to flange 602 and biostimulator body 217.

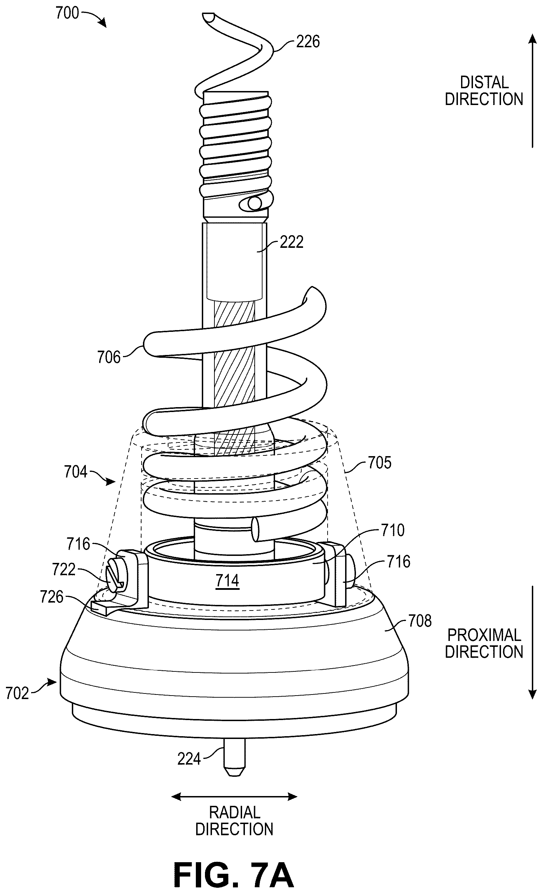

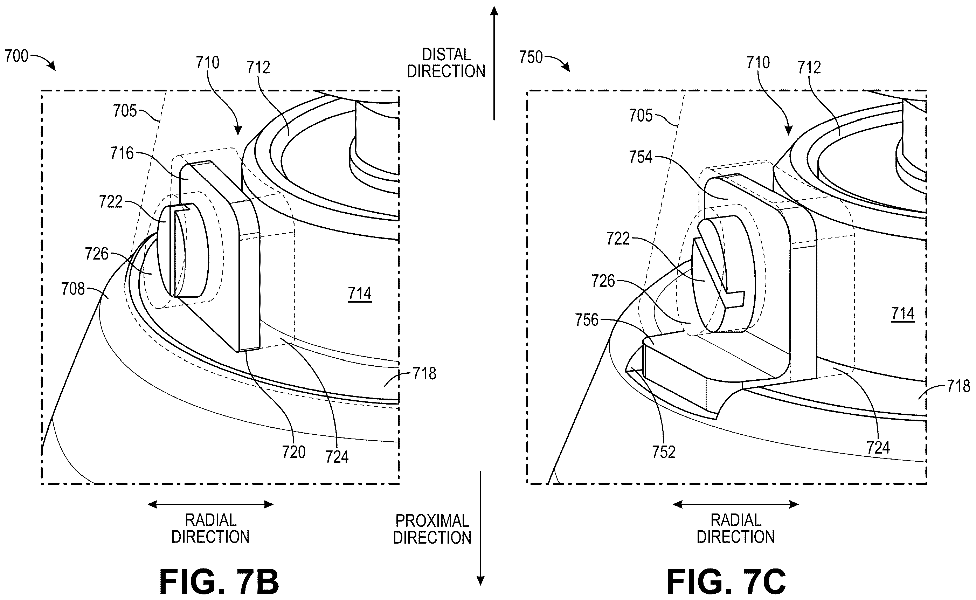

FIGS. 7A-7C together illustrate another embodiment of a distal fixation assembly 700 for a biostimulator 100. FIG. 7A is a side view, and FIG. 7B-7C are perspective views. Distal assembly 700 can include one or more components. The component(s) can include a flange 702, a fixation mount 704, or a fixation element 706. As in previous embodiments, lead body 222 is preferably centrally positioned in the flange 702, the fixation mount 704, and the fixation element 706.

Flange 702 is connected to the distal end 232 of body 217 and includes a feedthrough-e.g., a hole or other void in one embodiment-through which an electrical connection can be formed between electrical pacing element 226 and electrical circuitry within biostimulator 100. Flange 702 includes a base 708 and a hub 710 that surrounds and forms the feedthrough. In the illustrated embodiment hub 710 is centrally positioned on base 708, but in other embodiments it need not be centrally positioned. Hub 710 has an interior surface 712 and an exterior surface 714. Interior surface 712 is substantially at a first radial position and exterior surface 714 is substantially at a second radial location greater than the first radial location, so that the material between interior surface 712 and exterior surface 714 forms a hub sidewall. Interior surface 712 forms a hub lumen in which the feedthrough is formed.

As best shown in FIGS. 7B-7C, at least one tab 716 is formed on a distally-facing surface 718 of base 708 (i.e., a surface whose normal vector points substantially in a distal direction or has a non-zero component in the distal direction) and projects distally from the surface 718. The illustrated embodiment has two distally-projecting tabs 716, but other embodiments can have more or less tabs than shown. In one embodiment all tabs 716 can have the same size and shape, but in other embodiments all tabs 716 need not have the same size or shape. In embodiments with multiple tabs, the tabs can be uniformly spaced around the perimeter of base 708, but in other embodiments they need not be uniformly spaced. In one embodiment, distally-facing surface 718 includes a recess 720 (see FIG.7B) for each tab 716, and each tab 716 can be attached to distally-facing surface 718 by inserting each tab in its corresponding recess 720 and welding or otherwise fastening it in place. Each tab 716 includes a hole, e.g., a radial hole, therein to receive a fastener 722. As in FIGS. 6A-6B, the hole(s) can provide a first attachment mechanism of the distal fixation assembly 700. Furthermore, in the illustrated embodiment, the assembly lock can include a fastener 722, which may include a screw, but in other embodiments it can be a different type of fastener.

Fixation mount 704 is adapted to be attached to both flange 702 and fixation element 706 using tabs 716. In the illustrated embodiment, fixation mount 704 has an exterior surface 705 at frustoconical shape, but in other embodiments exterior surface 705 can have a different exterior shape than shown.

The proximal end of fixation mount 704 includes at least one recess 724 in a proximally-facing surface of the fixation mount 704 (i.e., a surface whose normal vector has non-zero proximal and radial components). The number of recesses 724 will usually correspond to the number of tabs 716, and the recesses 724 are sized and positioned so that each recess 724 will receive a corresponding tab 716 therein when the fixation mount 704 is attached to the flange 702. The exterior surface 705 of fixation mount 704 includes a hole 726 through which each fastener 722 can be inserted. The holes 726 can provide a second attachment mechanism to engage the first attachment mechanism provided by the holes in the flange 720. Each hole 726 extends through the sidewall of fixation mount 704 and will generally be aligned with a corresponding recess 724, so that when fastener 722 is inserted it goes into hole 726, through the sidewall of the fixation mount 704, and into a corresponding hole in tab 716. The fastener 722 can provide the assembly lock that engages and attaches the flange 702 to the fixation mount 704. In one embodiment hole 726 can have multiple different diameters: for instance, a small diameter to receive the fastener shank and a larger diameter to receive the fastener head, so that the fastener head can be recessed in exterior surface 705. After fastener 722 is inserted, a sealant such as epoxy can be used to fill any part of hole 726 not occupied by the fastener 722, thus creating a seal and also ensuring a smooth exterior surface 705.

Although not shown in these drawings, as in previous embodiments a distal part of the interior of fixation mount 704 has an interior surface in which is formed a helical groove with attributes such as pitch, which are matched to the pitch of fixation element 706, so that the fixation element 706 can be threaded into the helical groove. Once threaded into the helical groove, the fixation element 706 is secured to the interior of the fixation mount 704. By attaching the fixation element 706 to fixation mount 704, and attaching fixation mount 704 to hub 710, the fixation element 706 is attached to biostimulator body 217.

FIG. 7C illustrates an embodiment of a distal fixation assembly 750. Distal fixing assembly 750 is in most respects similar to distal fixation assembly 700; the primary difference is how tabs are attached to base 708. In distal fixation assembly 750, a radially-running groove 752 is cut into distally-facing surface 718. Tab 754 includes a radially-running base 756 that is substantially sized and shaped to fit into groove 752. As with tab 716, base 756, and hence tab 754, can be held in groove 752 by welding or some other method. In one embodiment of distal fixation assembly 750 all tabs 754 can have the same size and shape, but in other embodiments all tabs need not have the same size or shape. In embodiments with multiple tabs, the tabs can be uniformly spaced around the perimeter of base 708, but in other embodiments they need not be uniformly spaced. Once tabs 754 are in place, distal fixation assembly 750 functions substantially as described above for distal fixation assembly 700.

The above description of embodiments is not intended to be exhaustive or to limit the invention to the described forms. Specific embodiments of, and examples for, the invention are described herein for illustrative purposes, but various modifications are possible.

## Claims

1. An assembly (216, 300, 400, 500, 600, 700, 750) adapted to be attached to a biostimulator (100), the assembly (216, 300, 400, 500, 600, 700, 750) comprising:
a flange (218, 302, 402, 502, 602, 702) adapted to be attached to a distal end of the biostimulator (100), the flange (218, 302, 402, 502, 602, 702) including a base (308, 408, 508, 608, 708) adapted to be attached to the distal end of the biostimulator (100), and a hub (310, 410, 510, 610, 710) positioned on the base (308, 408, 508, 608, 708), the hub (310, 410, 510, 610, 710) having a proximal end (311), an interior surface (312, 412, 512, 612, 712), and an exterior surface (314, 414, 514, 614, 714), wherein the proximal end (311) of the hub (310, 410, 510, 610, 710) is attached to the base (308, 408, 508, 608, 708), and wherein the exterior surface (314, 414, 514, 614, 714) includes a first attachment mechanism (316, 416, 516, 616, 716);
a fixation mount (220, 304, 404, 504, 604, 704) adapted to be attached to the flange (218, 302, 402, 502, 602, 702), wherein the fixation mount (220, 304, 404, 504, 604, 704) includes a second attachment mechanism (324, 434, 534, 624, 726) to engage with the first attachment mechanism (316, 434, 534); and
an assembly lock (417, 625, 722) engages the first attachment mechanism (316, 416, 516, 616, 716) and the second attachment mechanism (324, 434, 534, 624, 726) to fasten the flange (218, 302, 402, 502, 602, 702) to the fixation mount (220, 304, 404, 504, 604, 704).

2. The assembly of claim 1 further comprising a fixation element (106, 306, 406, 506, 606, 706) positioned in the fixation mount (220, 304, 404, 504, 604, 704), wherein the flange (218, 302, 402, 502, 602, 702) and the fixation mount (220, 304, 404, 504, 604, 704), couple the fixation element (106, 306, 406, 506, 606, 706) to the distal end (217) of the biostimulator (100).

3. The assembly of claim 1 or 2, wherein:
the first attachment mechanism (316, 416, 516, 616, 716) comprises a first set of threads (316), and
the second attachment mechanism (324, 434, 534, 624, 726) comprises a second set of threads (324) complementary to the first set of threads (316).

4. The assembly of claim 3, wherein the assembly lock (417, 625, 722) includes an adhesive between the first set of threads (316) and the second set of threads (324).

5. The assembly of claim 1 or 2, wherein the first attachment mechanism (316, 416, 516, 616, 716) includes a groove (416) formed on the exterior surface (414) of the hub (410).

6. The assembly of claim 5, wherein the assembly lock (417, 625, 722) includes a retaining ring (417).

7. The assembly of claims 6, wherein the second attachment mechanism (324, 434, 534, 624, 726) includes a distally-facing lip (434), and wherein the distally-facing lip (434) engages the retaining ring (417).

8. The assembly of claim 6 or 7, wherein the retaining ring (417) is a C-ring.

9. The assembly of claim 1 or 2, wherein the first attachment mechanism includes a proximally-facing lip, wherein the second attachment mechanism includes a distally-facing lip, and wherein the distally-facing lip engages the proximally-facing lip to form the assembly lock.

10. The assembly of claim 1, wherein the first attachment mechanism (316, 416, 516, 616, 716) includes a radial hole (616) formed in an exterior sidewall of the hub (610), wherein the second attachment mechanism (324, 434, 534, 624, 726) includes a radial hole (624) formed in the fixation mount (604).

11. The assembly of claim 10, wherein the assembly lock (417, 625, 722) includes a pin (625) inserted into the radial hole (624) of the fixation mount (604) and the radial hole (616) of the flange (602).

12. The assembly of claim 1 or 2, wherein the first attachment mechanism (316, 416, 516, 616, 716) includes a tab (716) attached to the base (708), the tab (716) having a radial hole formed therein, and wherein the second attachment mechanism (324, 434, 534, 624, 726) includes a radial hole (726) formed in the fixation mount (704).

13. The assembly of claim 12, wherein the assembly lock (417, 625, 722) includes a screw (722) inserted into the radial hole (726) of the fixation mount (704) and the radial hole of the tab (716).

14. A biostimulator (100), comprising:
a body (217) having a proximal end (230) and a distal end (232); and
the fixation assembly (216, 300, 400, 500, 600, 700, 750) of any one of claims 1-13 attached to the distal end of the body (217).

15. A biostimulator system (200), comprising:
a biostimulator transport system (202) having a distal end; and
the biostimulator (100) of claim 14 mounted on the distal end of the biostimulator transport system (202).
